Europäisches Patentamt

European Patent Office

Office européen des brevets

(11) Numéro de publication: **0 280 610 B1**

# FASCICULE DE BREVET EUROPEEN

(45) Date de publication de fascicule du brevet: **21.10.92**  (51) Int. Cl.5: **C07K 5/06**, C07K 1/06

(21) Numéro de dépôt: **88400304.7**

(22) Date de dépôt: **10.02.88**

Le dossier contient des informations techniques présentées postérieurement au dépôt de la demande et ne figurant pas dans le présent fascicule.

(54) **Dipeptides, procédé de préparation et utilisation dans le dosage de protéases.**

(30) Priorité: **20.02.87 FR 8702259**

(43) Date de publication de la demande:
**31.08.88 Bulletin 88/35**

(45) Mention de la délivrance du brevet:
**21.10.92 Bulletin 92/43**

(84) Etats contractants désignés:
**DE**

(56) Documents cités:
EP-A- 0 000 330    EP-A- 0 074 787
EP-A- 0 085 255    CH-A- 616 912
FR-A- 2 471 411    GB-A- 2 130 221

CHEMICAL ABSTRACTS, vol. 92, 1980, page 742, résumé no. 129294c, Columbus, Ohio, US; M. BIENERT et al.: "Synthesis of substance P and of acylated partial sequences", & J. PRAKT. CHEM. 1979, 321(5), 721-40

CHEMICAL ABSTRACTS, vol. 83, 1975, pages 77-78, résumé no. 108713n, Columbus, Ohio, US; H. NIEDRICH et al.: "Action mechanism of peptides attacking smooth muscles. III. Effect of N-acylation upon the effectiveness of C-terminal partial sequences of eledoisin, physalemin, and the substance P at the guinea pig ileum", & ACTA BIOL. MED. GER. 1975, 34(3), 483-9

CHEMICAL ABSTRACTS, vol. 82, 1975, pages 667-668, résumé no. 140521p, Columbus, Ohio, US; & DD-A-107 947 (M. BIENERT et al.) 20-08-1974

(73) Titulaire: **SERBIO**
**30, Avenue Flachat**
**F-92600 Asnières(FR)**

(72) Inventeur: **Ouentin, Gérard**
**145, rue des Renouillers**
**F-92700 Colombes(FR)**

(74) Mandataire: **Clisci, Serge et al**
**S.A. FEDIT-LORIOT CONSEILS EN PROPRIETE INDUSTRIELLE 38, avenue Hoche**
**F-75008 Paris(FR)**

## Description

La présente invention concerne, en tant que produits industriels nouveaux, les composés dipeptides de formule I ci-après. Elle concerne également leur procédé de préparation et leur utilisation dans le domaine du dosage de protéases et de peptidases , en particulier en tant que substrats dans le domaine du dosage quantitatif d'enzymes de la classe E.C. 3.4.4 (à présent nouvelle classe "E.C. 3.4.21", selon l'ouvrage "Enzyme Nomenclature", Elsevier Scientific Publishing Company, Amsterdam 1973, page 238 et suivantes).

## ART ANTERIEUR

On sait que les enzymes de la classe sus-visée sont des substances qui scindent les liaisons amides du squelette protéique ou peptidique du côté du groupe carboxyle des restes Arg, Lys, Orn et His. Il s'agit là d'un mécanisme de clivage bien connu de l'homme du métier et qui est amplement exemplifié dans les documents antérieurs cités ci- après.

On sait que l'on a déjà proposé dans le passé des substrats spécifiques pour le dosage d'enzymes de ladite classe tels que notamment la thrombine (E.C. 3.4.21.5), la plasmine (E.C. 3.4.21.7), le Facteur Xa (E.C. 3.4.21.6) et la trypsine (E.C. 3.4.31.4), lesdits substrats étant des substances de formule brute :

Xo - Ao - Yo    (Io)

dans laquelle Xo représente un groupe protecteur et le cas échéant un atome d'hydrogène; Yo est un groupe de marquage, notamment un reste radioactif ou un reste capable de conférer, avant ou (mieux) après clivage, une coloration ou une fluorescence; et, Ao est un reste de monoaminoacide ou de polyaminoacide et de préférence un reste tri- ou tétrapeptidique.

Le groupe protecteur Xo est fixé sur l'extrémité N-terminale de la chaîne Ao et représente de façon classique dans la littérature antérieure un groupe acétyle, benzoyle, t-butyloxycarbonyle, benzyloxycarbonyle, succinyle, tosyle ou analogue.

Historiquement, les premiers substrats qui ont été proposés sont ceux dans lesquels Ao représentait un reste de monoaminoacide. Voir à cet effet, l'article de ERLANGER et al., Arch. Biochem. Biophys., $\underline{95}$, 271 (1961) qui décrit les premiers substrats du type

$C_6 H_5 CO$-D,L-Arg-pNA

Les dérivés de formule Io où Ao est un reste de monoaminoacide ne se sont pas révélés particulièrement intéressants en tant que substrats d'enzymes, essentiellement par défaut de sélectivité. Néanmoins, on sait que l'acide γ-glutamyl-p-nitroanilide-3-carboxylique a été proposé dans FR-A- 2 208 886 comme substrat chromogène de la γ-glutamyl transpeptidase (E.C. 2.3.2.2), et que, dans FR-A-2 546 163 ont été proposés (i) les PHA-L-Phe-Yo et PHA-L-Tyr-Yo (où PHA désigne un reste polyhydroxyalkyle) pour le dosage de la chymotrypsine (E.C. 3.4.31.1), et (ii) les PHA-L-Arg-Yo et PHA-L-Lys-Yo pour le dosage de la trypsine (E.C.3.4.31.4).

Pour pallier les difficultés des substrats monoaminoacides, on sait que l'on a proposé des composés de formule Io, où Ao représente plus particulièrement un reste tri- ou tétrapeptidique et Yo un groupe chromogène scindable ou clivable par hydrolyse enzymatique, pour le dosage des enzymes de la classe susvisée. Le nombre de publications concernant les substrats tétrapeptidiques et surtout tripeptidiques est considérablement supérieur à celui relatif aux substrats de monoaminoacide. Pour information, des tripeptides et des tétrapeptides sont décrits dans les documents FR-A-2 546 163 précité, FR-A- 2 372 798, EP-A-0 004 256, US-A- 4 508 644, US-A- 4 448 715, FR-A- 2 317 280 et FR-A- 2 459 226.

On sait notamment de l'exposé de BARBIER présenté lors du 10ème Congrès National des Biologistes des Hôpitaux Généraux tenu à Hyères les 7-9 octobre 1981, que les composés tétrapeptidiques et surtout tripeptidiques de formule $I_o$ étaient les plus intéressants eu égard à leur spécificité vis-à-vis d'enzymes protéolytiques.

En particulier ont été commercialisés avec succès les tripeptides suivants :

H-D-Abu-L-CHA-L-Arg-pNA,2AcOH (cf. exemple 41 de US-A-4 428 874) en tant que substrat de la plasmine,

H-D-CHG-L-Abu-L-Arg-pNA, 2AcOH (cf. exemple 71 de US-A-4 428 874) en tant que substrat de la thrombine, et $CH_3 SO_2$-D-Leu-Gly-L-Arg-pNA, AcOH (selon notamment US-A-4 440 678) en tant que substrat du facteur Xa.

EP 0 280 610 B1

On sait également que des tri- et tétrapeptides chromogènes présentant sur l'extrémité N-terminale de la chaine peptidique un groupe ω-méthoxycarbonylalkylènecarbonyle ou ω-éthoxycarbonylalkylènecarbonyle (où le fragment alkylènecarbonyle comporte 2 à 4 atomes de carbone) ont été proposés dans EP-A-0 128 118 qui décrit spécifiquement les EtO -CO-CH$_2$-CO-Lys ($\epsilon$-Cbo)-Gly-Arg-pNA, AcOH (cf. exemple 14) et MeO-CO-CH$_2$CH$_2$-CO-Lys ($\epsilon$-Cbo)-Gly-Arg-pNA, AcOH (cf. exemple 19). Or, il se trouve que, dans la série des tri- et tétrapeptides, le remplacement d'un atome d'hydrogène de l'extrémité N-terminale NH$_2$ par un groupe oxycarbonylalkylènecarbonyle entraîne une diminution systématique de l'activité (voir à cet effet les résultats d'essais comparatifs consignés dans le tableau III ci-après).

On sait par ailleurs qu'il existe un très grand nombre de publications ayant trait à l'obtention de composés dipeptidiques. Pratiquement toutes ces publications présentent les composés dipeptidiques, qu'elles décrivent, en tant qu'intermédiaires de synthèse des tri- et tétrapeptides susvisés.

Ponctuellement, le nombre de documents mentionnant l'utilisation de dipeptides comme substrats est très faible, à savoir :

(i) US-A-4 217 269 qui décrit des phénoxyacétyldipeptides chromogènes, en tant que substrats de la thrombine et de la trypsine, notamment les PhO-CH$_2$CO-L-Pro-L-Arg-pNA, PhO-CH$_2$CO-L-Pip-L-Arg-pNA, PhO-CH$_2$CO-L-Aze-L-Lys-pNA, et PhO-CH$_2$CO-L-Pro-L-Orn-pNA;

(ii) FR-A-2 471 411 qui propose en tant que substrats les dipeptides de formule

HO-CH(R$_1$)-CO-B$_1$-B$_2$-R$_2$

où

R$_1$     est H, alkyle en C$_1$-C$_4$ ou Bzl,

B$_1$     est Gly, Ala, Val, Leu, Pro, Ile, Ser, Thr, Asp, Asn, Gln, Glu, Lys, hydroxyglycyle, His, Arg, Phe, Tyr, Trp, Cys, Met ou Pip,

B$_2$     est L-Arg ou L-Lys, et

R$_2$     est un reste chromogène ou fluorogène,

d'une part, et décrit spécifiquement en tant que substrats, pour le dosage de l'antithrombine III, les dipeptides de formule :

X$_0$-L-Pro-L-Arg-pNA

où X$_o$ est un groupe $\alpha$-hydroxyacyle choisi parmi les L- et D-$\alpha$-hydroxy-$\beta$-phénylpropionyle, L- et D-$\alpha$-hydroxyisocaproyle, et, glycoyle, d'autre part;

(iii) US-A-4 448 715 précité qui décrit spécifiquement des tripeptides en tant que substrats de la kallikréine plasmatique et englobe dans sa formule générale des dipeptides de structure :

X$_0$-L-Phe-L-Arg-Yo

(où Yo est défini comme ci-dessus et X$_o$ représente un groupe cycloalkylcarbonyle dans lequel le reste cycloalkyle comprend de 3 à 6 atomes de carbone) qui ne sont pas exemplifiés; de même des dipeptides sont inclus dans les formules générales proposées par FR-A-2 546 163 précité et DE-A-3 108 322, mais ne sont pas spécifiquement exemplifiés dans ces documents.

**OBJET DE L'INVENTION**

Suivant l'invention, on préconise de nouveaux composés dipeptidiques de formule I ci-après qui (i) diffèrent des dipeptides antérieurement connus comme intermédiaires de synthèse ou substrats par la nature du groupe protecteur Q de l'extrémité N-terminale, qui comporte 2 groupes carbonyle et plus précisément une structure linéaire ω-oxycarbonylalkylènecarbonyle :

-O-CO-CH$_2$-CO-

et (ii) sont utiles en tant que substrats vis-à-vis des enzymes de la classe E.C.3.4.21 susvisée et de l'ancienne classe E.C.3.4.4.

On vient de trouver de façon surprenante que ledit groupe protecteur Q, qui a pour formule RO-CO-CH$_2$-CO (où R est défini comme indiqué ci-après) et qui est partiellement exemplifié dans EP-A-0 128 118 précité, ne provoque pas dans la série des dipeptides suivant l'invention la baisse d'activité observée pour les séries des tri- et tétrapeptides. Par ailleurs un tel groupe Q n'apparaît pas dans les dipeptides

3

intermédiaires décrits dans EP-A-0 128 118.

Les composés dipeptidiques suivant l'invention sont notamment intéressants en tant que substrats pour le dosage quantitatif de la thrombine, de la plasmine et de la Protéine C. On a également trouvé qu'ils sont en outre particulièrement intéressants eu égard à leur spécificité vis-à-vis de la Protéine C.

Les nouveaux dérivés dipeptidiques suivant l'invention sont caractérisés en ce qu'ils sont choisis parmi l'ensemble comprenant :

(i) les composés de formule :

$$Q\text{-}A_1\text{-}A_2\text{-}R_1 \qquad (I)$$

dans laquelle :

Q est un reste $RO\text{-}CO\text{-}CH_2\text{-}CO$ où

R représente l'atome d'hydrogène, un groupe alkyle en $C_1\text{-}C_4$, un groupe phényle éventuellement substitué par un ou plusieurs groupes notamment $CH_3$ et $OCH_3$, un groupe 3,4-méthylènedioxyphényle, un groupe benzyle éventuellement substitué par un ou plusieurs groupes, notamment $CH_3$ et $OCH_3$, un groupe 3,4-méthylènedioxybenzyle, ou, un groupe tosylméthyle;

$R_1$ qui est un reste amino de formule $NH\text{-}R'_1$ constitue un marqueur clivable du groupe $A_2$ par hydrolyse enzymatique, le groupe $R'_1$ intervenant comme support du moyen de marquage;

$A_1$ est un reste de monoaminoacide choisi parmi l'ensemble comprenant les restes d'aminoacides non-basiques;

$A_2$ est un reste de monoaminoacide choisi parmi l'ensemble comprenant les restes d'$\alpha$-aminoacides basiques; et

(ii) leurs sels d'addition.

## ABREVIATIONS

Dans la présente description, on a utilisé les abréviations suivantes par commodité :

- pour les restes d'aminoacide

| | | |
|---|---|---|
| Acp | = | $\epsilon$-aminohexanoyle (ou 6-aminocaproyle) |
| ACC | = | 1-amino-cyclohexane-1-carbonyle |
| Aib | = | 2-aminoisobutyryle (ou 2-méthylalanyle) |
| Ala | = | $\alpha$-alanyle |
| bAla | = | $\beta$-alanyle |
| Arg | = | arginyle |
| Asn | = | asparaginyle |
| Asp | = | $\alpha$-aspartyle |
| bAsp | = | $\beta$-aspartyle |
| ATC | = | thiazolidine-4-carbonyle (ou thioprolyle) |
| Aze | = | azétidine-2-carbonyle |
| Abu | = | 2-aminobutyryle (ou $\alpha$-amino-n-butyryle) |
| 4Abu | = | 4-aminobutyryle (ou $\gamma$-amino-n-butyryle) |
| CHA | = | 3-cyclohexylalanyle |
| CHG | = | $\alpha$-cyclohexylglycyle |
| CHT | = | 3-(4-hydroxycyclohexyl)alanyle |
| Cle | = | cyclocleucyle (ou 1-amino-cyclopropane-1-carbonyle) |
| Cys | = | cystéyle |
| Dbu | = | 2,4-diaminobutyryle |
| $\pi$Dbu | = | pyrodiaminobutyryle |
| Gln | = | glutamyle |
| Glu | = | glutaminyle |
| $\gamma$-Glu | = | $\gamma$-glutaminyle (ou $\gamma$-Glu) |
| Gly | = | glycyle |
| His | = | histidyle |
| 4Hyp | = | 4-hydroxyprolyle (ou 4-hydroxy-2-pyrrolidine-carbonyle |
| 3Hyp | = | 3-hydroxyprolyle (ou 3-hydroxy-2-pyrrolidine-carbonyle) |
| Ile | = | isoleucyle |
| Leu | = | leucyle |
| Lys | = | lysyle |

| Met | = méthionyle |
|---|---|
| Nle | = norleucyle |
| Nva | = norvalyle |
| Orn | = ornithinyle |
| Phe | = phénylalanyle |
| Phg | = $\alpha$-phénylglycyle |
| Pip | = pipécolinoyle |
| Pro | = prolyle |
| Pyr | = pyroglutaminyle (ou 2-pyrrolidone-5-carbonyle) |
| MeGly | = sarccsyle (ou Sar) |
| Ser | = séryle |
| Thr | = thréonyle |
| Tyr | = tyrosyle |
| Val | = valyle |

- pour les autres abréviations :

| Ac | = acétyle |
|---|---|
| AcOH | = acide acétique |
| Adoc | = adamantyloxycarbonyle |
| Aoc | = t-amyloxycarbonyle |
| Boc | = t-butyloxycarbonyle |
| Bz | = benzoyle |
| Bzl | = benzyle |
| Cbo | = carbobenzoxy |
| CCM | = chromatographie sur couche mince |
| o-Cl-pNA | = o-chloro-p-nitroanilide (ou o-chloro-p-nitroanilino) |
| DCCI | = dicyclohexylcarbodiimide |
| DCHU | = dicyclohexylurée |
| DMF | = diméthylformamide |
| Et | = éthyle |
| $Et_3N$ | = triéthylamine |
| Foc | = furfuryloxycarbonyle |
| HMPT | = N,N,N',N',N'',N''- hexaméthylphosphorotriamide |
| HOBT | = 1-hydroxybenzotriazole |
| HPLC | = chromatographie liquide de haute performance |
| H-TFA | = acide trifluoroacétique |
| Iboc | = isobornyloxycarbonyle |
| Me | = méthyle |
| MM | = $CH_3O-CO-CH_2-CO-$ ( i.e. méthyloxymalonyle, groupe protecteur suivant l'invention). |
| nkat | = nanokatal (1 nkat est l'activité enzymatique d'un enzyme qui hydrolyse 1 nanomole de son substrat par seconde dans les conditions standard) |
| OD | = densité optique |
| pNA | = p-nitroanilide (ou p-nitroanilino) |
| Ph | = phényle |
| RT | = température ambiante (15-20 °C) |
| TEA | = triéthanolamine |
| Tos | = p-toluènesulfonyle (ou tosyle) |
| UNIH | = unité enzymatique standardisée de l'"US National Institute of Health" |
| Z | = benzyloxycarbonyle |
| Z (p-Cl) | = p-chlorobenzyloxycarbonyle |
| Z(p-OMe) | = p-méthoxybenzyloxycarbonyle |

## DESCRIPTION DETAILEE DE L'INVENTION

Parmi les groupes alkyle en $C_1$-$C_4$ inclus dans les définitions de R, qui conviennent selon l'invention, on peut mentionner notamment les groupes $CH_3$, $CH_2CH_3$, $CH_2CH_2CH_3$, $CH(CH_3)_2$, $C(CH_3)_3$ et $CH(CH_3)$-$CH_2CH_3$.

Parmi les groupes benzyle substitués qui entrent dans la définition de R on peut notamment citer les groupes 3-méthylbenzyle, 3,5-diméthylbenzyle , 4-méthylbenzyle, 4-méthoxybenzyle, 3,4-diméthoxybenzy-

5

le, 2,4,6-triméthoxybenzyle et 3,4-méthylènedioxybenzyle.

Parmi les groupes phényle substitués, qui entrent dans la définition de R, on peut notamment mentionner les groupes o-,m- et p-tolyle, xylyle, 2-, 3- et 4-méthoxyphényle, 3,5-diméthoxyphényle. 3,4-diméthoxyphényle, 2,4-diméthoxyphényle, 3,4,5-triméthoxyphényle. 2,4,6-trimethoxyphenyle, 3,4-méthylènedioxyphényle, 3-méthoxy-4-méthylphényle et 4- méthoxy-3-méthylphényle.De façon avantageuse, on préfère surtout suivant l'invention que R = $CH_3$.

$A_1$ est un reste de monoaminoacide non basique. Il provient d'un aminoacide naturel ou synthétique ayant un pH inférieur ou égal à 7 ou d'un aminoacide ayant un pH supérieur à 7 mais convenablement substitué pour bloquer essentiellement son caractère basique et atteindre un pH inférieur ou égal à 7.

$A_1$ englobe donc:

1°)les restes d'aminoacides naturels ou synthétiques qui comprennent une seule fonction acide et une seule fonction basique,

2°) les restes d'aminoacides naturels ou synthétiques qui comprennent une seule fonction basique et plus d'une fonction acide et dans lesquels chaque fonction acide latérale (n'entrant pas ou n'intervenant pas dans la liaison peptidique) est susceptible d'être bloquée notamment sous forme amide ou ester et

3°) les restes d'aminoacides naturels ou synthétiques qui comprennent une seule fonction acide et plus d'une fonction basique et dans lesquels chaque fonction basique latérale est convenablement substituée pour bloquer et supprimer essentiellement son caractère basique.

Bien entendu, les restes d'aminoacides sus-visés, qui sont notamment énumérés dans le chapitre abréviation ci-dessus, peuvent comporter des groupes hydroxyle (OH) ou thiol (SH) latéraux qui sont susceptibles d'être bloqués,le cas échéant, par un groupe protecteur éther ou ester.

Conviennent en particulier pour $A_1$, les restes provenant d'aminoacides :

- naturels non-basiques dits "neutres" tels que notamment Ala, bAla, Cys, Gln, Gly, 4Hyp, 3Hyp, Leu, Met, Nle, Nva, Phe, Pro, MeGly, Ser, Thr, Tyr, Val, où le groupe OH latéral des restes 4Hyp, 3Hyp, Ser, Thr, Tyr est protégé ou non par un groupe protecteur éther ou ester, et où le groupe SH latéral du reste Cys est protégé ou non par un groupe protecteur thioéther ou thioester;
- naturels acides tels que notamment Asp, bAsp, Glu, γ-Glu, dans lesquels la fonction acide latérale est libre ou bloquée sous forme ester, notamment sous forme d'ester benzylique ou t-butylique;
- naturels initialement basiques dans lesquels la ou les fonctions basiques latérales n'entrant pas dans la liaison peptidique sont bloqués par un groupe convenable éliminant pratiquement le caractère basique tel que Boc, Cbo etc, lesdits restes d'aminoacide étant notamment les restes Arg, Lys, His et Orn convenablement bloqués notamment par un groupe Cbo;
- synthétiques non-basiques dits "neutres" tels que notamment ACC, Acp, Aib, ATC, Aze, Abu, 4Abu, CHA, CHG, CHT (où le groupe OH latéral est protégé le cas échéant sous forme éther ou ester), Cle, πDbu, Phg, Pip, Pyr;
- synthétiques initialement basiques dans lesquels chaque fonction basique latérale est protégée par un groupe convenable éliminant substantiellement le caractère basique, notamment le reste Dbu où la fonction basique latérale est bloquée par un groupe tel que Cbo.

Parmi les restes d'aminoacides synthétiques qui conviennent également, on peut notamment mentionner (i) les restes d'aminoacides cycloaliphatiques où le groupe amino et le groupe carboxy sont situés sur le même atome de carbone cyclique (tels que Cle susvisé) ou sur deux atomes de carbone cycliques différents et (ii) les restes d'aminoacides aromatiques tels que notamment les restes o-, m- et p-aminobenzoyle, et, les restes d'aminoacides aralkyliques tels que notamment les p-aminobenzylcarbonyle, m-aminobenzylcarbonyle.

De façon avantageuse le reste A, selon l'invention sera un reste d'α-aminoacide tel que ACC, Aib, Ala, ATC, Aze, Abu, CHA, CHG, CHT, Cle, Gly, 4Hyp, 3Hyp, Ile, Leu, Met, Nle, Nva, Phe, Pip, Pro, MeGly, Ser, Thr, Tyr, Val, un reste bAla, ou un reste CHT, 4Hyp, 3Hyp, Ser, Thr, Tyr, dans lequel le groupe latéral OH est protégé par un groupe éther ou ester.

Le reste $A_1$ comprend donc des restes d'aminoacides tels que Aib, Cle, Gly, MeGly et 4Abu dépourvus d'atome de carbone asymétrique, et des restes d'aminoacides présentant un atome de carbone asymétrique.

Lorsque l'aminoacide présente un atome de carbone asymétrique, le reste $A_1$ est, de préférence, de configuration L car les dipeptides de formule I dans lesquels $A_1$ est un reste de configuration D sont en général inactifs ou peu actifs en tant que substrats.

Le groupe $A_1$ selon l'invention sera de préférence un groupe Aib, L-Ala, bAla, L-ATC, L-Aze, L-Abu, L-CHA, L-CHG, L-CHT, Cle, Gly, L-4Hyp, L-3Hyp, L-Ile, L-Leu, L-Nle, L-Nva, L-Phe, L-Pip, L-Pro, MeGly, L-Ser, L-Thr, L-Tyr, L-Val ou un reste L-CHT, L-4Hyp, L-3Hyp, L-Ser, L-Thr ou L-Tyr dans lequel le groupe OH latéral est protégé par un groupe éther ou ester.

A$_2$ est le reste d'un α-aminoacide basique. Il provient d'un monoaminoacide ayant un pH supérieur à 7, c'est-à-dire comportant une fonction acide COOH et, en plus de la fonction basique en α, au moins une fonction basique latérale. Dans A$_2$ ladite fonction basique latérale n'est pas bloquée. Parmi les restes d'α-aminoacides qui conviennent, on peut mentionner notamment les restes L-Dbu, L-Arg, L-Lys, L-His et L-Orn. Les restes préférés sont des restes d'α-L-aminoacides, notamment L-Arg, L-Lys, L-His et L-Orn, le reste le plus intéressant selon l'invention étant L-Arg.

Le groupe marqueur aminé R$_1$ est bien connu dans la technique des dosages biologiques et microbiologiques, voir à cet effet le document US-A-4 448 715 précité. Il peut notamment être choisi parmi les groupes NH-R'$_1$ qui (i) induisent une modification de coloration, (ii) induisent une modification de fluorescence ou (iii) comportent au moins un élément radioactif. Convient au but de l'invention tout groupe amino NH-R'$_1$ donnant pendant ou après la réaction enzymatique un signal susceptible d'être amplifié pour être détecté (par exemple par mesure de la densité optique sous une longueur d'onde donnée, ou encore par mesure de la radioactivité).

Parmi les groupes fluorescents entrant dans la définition de R$_1$, on peut notamment citer les 4-méthylcoumaryl-7-amino, 4-trifluorométhylcoumaryl-7-amino et analogues.

Le groupe R$_1$ peut également représenter un groupe comportant un élément radioactif, par exemple un groupe anilino ou benzylamino marqué par un radioisotope $^{14}$C ou $^{3}$H.

Le groupe R'$_1$ que l'on préfère suivant l'invention est un groupe chromogène, de façon typique un groupe nitrophényle (dans lequel le reste phényle est susceptible d'être substitué par un groupe COOH, F, Cl, Br, CH$_3$, OCH$_3$, CN, CF$_3$, et/ou SO$_3$H), ou un groupe fluorescent, de façon typique un groupe naphtyle (dans lequel le reste naphtyle est susceptible d'être substitué par un groupe OCH$_3$, COOH, SO$_3$H ou CH$_3$), un groupe pyrimidinyle, benzimidazolyle, triazinyle, indazolyle, pyrènyle, coumaryle, quinolyle etc.

Le groupe R$_1$ que l'on préfère suivant l'invention est pNA, qui convient d'une manière générale à la détermination quantitative des enzymes protéolytiques susvisés et est particulièrement adapté à la détermination de la Protéine C et de la plasmine.

Les sels d'addition suivant l'invention sont essentiellement des sels d'addition d'acide obtenus par réaction d'un composé de formule I avec un acide minéral ou organique.

Le meilleur mode de mise en oeuvre de l'invention consiste à utiliser comme substrat un composé dipeptide, dans lequel R est CH$_3$, R$_2$ et R$_3$ sont H, et, n vaut 1, et répondant à la formule :

CH$_3$O-CO-CH$_2$-CO-A$_1$-Arg-pNA    (II)

où A$_1$ est choisi parmi les restes Acp, Aib, L-Ala, bAla, L-ATC, L-Aze, L-Abu, L-CHA, L-CHG, L-CHT, Gly, L-4Hyp, L-3Hyp, L-Ile, L-Leu, L-Nle, L-Nva, L-Phe, L-Pip, L-Pro, MeGly, L-Ser, L-Thr, L-Tyr et L-Val et les restes L-CHT, L-4Hyp, L-3Hyp, L-Ser, L-Thr et L-Tyr dans lesquels le groupe OH latéral est protégé sous forme éther ou ester.

Les composés de formule II ont l'avantage de présenter une grande affinité pour l'eau à la différence des dérivés peptidiques antérieurement connus qui sont peu solubles ou peu dispersables dans l'eau. En raison de la faible affinité pour l'eau des peptides antérieurement connus, il est quelquefois nécessaire de leur adjoindre un solvant organique afin de pouvoir les utiliser. Les systèmes comprenant des solvants mixtes (notamment du type eau-solvant organique) ne sont guère compatibles d'une manière générale avec les milieux biologiques : ils entraînent soit une diminution de l'activité du substrat, soit encore dans certains cas une détérioration de l'enzyme que l'on veut doser. De plus, la faible affinité pour l'eau de ces substrats entraîne une diminution de la sensibilité des méthodes de dosage des enzymes. L'utilisation des composés de formule II permet de pallier avantageusement les inconvénients sus-énoncés.

De façon non limitative on a consigné dans le tableau I ci-après un certain nombre de composés dipeptides suivant l'invention notés "Ex" avec des peptides de référence notés "A1-A4", et, des peptides de comparaison notés "B1-B4" comportant le groupe protecteur terminal CH$_3$O-CO-CH$_2$-CO selon l'invention et "C1-C4" sans ledit groupe protecteur.

## TABLEAU I

### Q-A$_1$-A$_2$-pNA, xHX

| PRODUIT | N° DE CODE | Q | A$_1$ | A$_2$ | xHX |
|---|---|---|---|---|---|
| EX 1 | SQ 41 | MM | L-Pro | L-Arg | H-TFA |
| EX 2 | SQ 57 | MM | L-Val | L-Arg | H-TFA |
| EX 3 | SQ 58 | MM | L-Ala | L-Arg | H-TFA |
| EX 4 | SQ 59 | MM | L-Phe | L-Arg | H-TFA |
| EX 5 | SQ 60 | MM | L-Ser(Bzl) | L-Arg | H-TFA |
| EX 6 | SQ 61 | MM | bAla | L-Arg | H-TFA |
| EX 7 | SQ 62 | MM | L-Leu | L-Arg | H-TFA |
| EX 8 | SQ 63 | MM | L-Nle | L-Arg | H-TFA |
| EX 9 | SQ 64 | MM | L-Nva | L-Arg | H-TFA |
| EX 10 | SQ 65 | MM | L-4Hyp | L-Arg | H-TFA |
| EX 11 | SQ 66 | MM | Gly | L-Arg | H-TFA |
| EX 12 | SQ 67 | MM | L-ATC | L-Arg | H-TFA |
| EX 13 | SQ 68 | MM | Aib | L-Arg | H-TFA |
| EX 14 | SQ 69 | MM | L-Abu | L-Arg | H-TFA |
| EX 15 | SQ 70 | MM | L-Thr(Bzl) | L-Arg | H-TFA |
| EX 16 | SQ 73 | MM | Acp | L-Arg | H-TFA |
| EX 17 | – | MM | L-CHA | L-Lys | H-TFA |
| EX 18 | – | MM | L-Lys($\varepsilon$-Cbo) | L-Arg | AcOH |

8

## TABLEAU I (suite)

| PRODUIT | N° DE CODE | Q | $A_1$ | $A_2$ | xHX |
|---|---|---|---|---|---|
| EX 19 | – | (1) | L-Val | L-Arg | AcOH |
| EX 20 | – | (2) | L-Leu | L-Orn | AcOH |
| EX 21 | – | (2) | L-CHG | L-Arg | AcOH |
| EX 22 | – | (1) | L-CHA | L-Arg | AcOH |
| EX 23 | – | MM | Acp | L-Lys | AcOH |
| EX 24 | – | MM | L-Pro | L-His | AcOH |
| $A_1$ | 34.47 | H | D-CHG-L-Abu | L-Arg | 2AcOH |
| $A_2$ | 31.39 | $CH_3SO_2$ | D-Leu-Gly | L-Arg | AcOH |
| $A_3$ | 30.41 | H | D-Abu-L-CHA | L-Arg | 2AcOH |
| $A_4$ | 65.25 | H | D-Lys($\varepsilon$-Cbo)-L-Pro | L-Arg | 2AcOH |
| $B_1$ | SQ 72 | MM | Gly-L-Pro | L-Arg | H-TFA |
| $C_1$ | – | H | Gly-L-Pro | L-Arg | 2 H-TFA |
| $B_2$ | SQ 71 | MM | Gly-L-Pip | L-Arg | H-TFA |
| $C_2$ | – | H | Gly-L-Pip | L-Arg | 2 H-TFA |
| $B_3$ | SQ 56 | MM | D-Pro-L-Pro | L-Arg | H-TFA |
| $C_3$ | 55.10 | H | D-Pro-L-Pro | L-Arg | 2 H-TFA |
| $B_4$ | SQ 52 | MM | D-Nle-L-CHA | L-Arg | H-TFA |
| $C_4$ | 33 08 | H | D-Nle-L-CHA | L-Arg | 2 H-TFA |

## Notes

(1) $pCH_3C_6H_4SO_2CH_2O-CO-CH_2-CO$

(2) $pMeOC_6H_4O-CO-CH_2CO$

Les composés dipeptides de formules I et II ci-dessus peuvent être préparés selon une méthode connue en soi par application de mécanismes réactionnels classiques. Suivant l'invention on préconise un procédé de préparation qui est caractérisé en ce qu'il comprend :

1°) la réaction d'un $\alpha$-aminoacide N-protégé par un groupe protecteur temporaire approprié Z, de formule:

$Z_1$-$A_2$-OH     (III)

où $A_2$ est défini comme ci-dessus, avec un isocyanate, correspondant au groupe marqueur $R_1$, de formule :

$O = C = N\text{-}R'_1$     (IV)

pour obtenir un composé de formule :

$Z_1\text{-}A_2\text{-}R_1$     (V)

2°) la soumission du composé V ainsi obtenu à une réaction d'acidolyse au moyen d'un mélange HBr/AcOH pour éliminer le groupe protecteur $Z_1$ et obtenir le dérivé de formule :

$H\text{-}A_2\text{-}R_1$     (VI)

3°) la réaction du dérivé VI ainsi obtenu avec un aminoacide N-protégé de formule :

$Z_2\text{-}A_1\text{-}OH$     (VII)

où $A_1$ est défini comme indiqué ci-dessus et $Z_2$ est un groupe protecteur temporaire, pour obtenir par condensation un dipeptide protégé de formule :

$Z_2\text{-}A_1\text{-}A_2\text{-}R_1$     (VIII)

4°) la soumission dudit dipeptide N-protégé de formule VIII ainsi obtenu à une réaction d'acidolyse au moyen de l'acide trifluoroacétique pour éliminer le groupe protecteur $Z_2$ et donner un dipeptide de formule :

$H\text{-}A_1\text{-}A_2\text{-}R_1$     (IX)

et,

5°) la réaction du dipeptide de formule IX ainsi obtenu avec un composé de formule :

$R\text{-}O\text{-}CO\text{-}CH_2\text{-}CO\text{-}T$     (X)

où R est défini comme indiqué ci-dessus et T représente OH, F, Cl ou Br (l'atome d'halogène préféré étant le chlore) pour obtenir le composé de formule I attendu.

Le groupe protecteur temporaire $Z_1$ qui intervient au stade 1° est ici un groupe protecteur classique bien connu de l'homme de l'art, par exemple Boc, Aoc, Adoc, Foc, Iboc, Z, Z(p-Cl), Z(p-OMe) ou analogue. Le groupe protecteur temporaire $Z_2$ est un groupe protecteur classique de l'extrémité N-terminale, ici ce groupe est identique ou analogue à $Z_1$.

Le stade 5° comprend la réaction du dipeptide de formule IX avec un halogènure d'acide :

$R\text{-}O\text{-}CO\text{-}CH_2\text{-}CO\text{-}Hal$     (XI)

où Hal représente un halogène F, Cl, ou Br, ou avec un acide :

$R\text{-}O\text{-}CO\text{-}CH_2\text{-}CO\text{-}OH$     (XII)

De façon avantageuse la réaction IX + XI → I est mise en oeuvre dans un solvant inerte tel que notamment le DMF en présence d'une base en excès agissant comme co-solvant et accepteur de protons , notamment $Et_3N$, avec un rapport molaire IX/XI inférieur ou égal à 1/2.

De façon avantageuse la réaction IX + XII est effectuée dans un solvant inerte tel que notamment le DMF en présence d'une base en excès intervenant comme co-solvant et accepteur de protons, notamment $Et_3N$, de HOBT et de DCCI, à une température voisine de 0°C pendant au moins 1 heure puis à RT pendant au moins 24 heures, le rapport molaire IX/XII étant compris entre 1/1 et 1/1,5.

De façon préférée, on utilisera dans la réaction IX + XI → I un rapport molaire IX/$Et_3$N inférieur ou égal à 1/2,7 et dans la réaction IX + XII → I un rapport molaire IX/$Et_3$N de l'ordre 1/1,1, un rapport molaire IX/HOBT de l'ordre de 1/2 et un rapport molaire IX/DCCI de l'ordre de 1/1,18 à 1/1,20. Dans cette dernière réaction le couplage se fait par une activation in situ de la fonction acide en présence de DCCI et HOBT, HOBT étant un agent de couplage non racémisant.

D'autres avantages et caractéristiques de l'invention seront mieux compris à la lecture qui va suivre d'exemples de préparation et d'essais comparatifs, l'ensemble de ces éléments n'étant pas limitatif mais donné à titre d'illustration.

**PREPARATION I**

Obtention de MM-L-4Hyp-L-Arg-pNA, H-TFA

(Exemple 10 : No de code : SQ 65)

a) Z-L-Arg-pNA, HCl

On dissout 13 g (41 mmoles) de Z-L-Arg-OH, HCl (préalablement séché sur $P_2O_5$) dans 100 ml de HMPT anhydre à RT. On additionne a RT 5.7 ml de $Et_3N$ et ensuite graduellement 82 mmoles d'isocyanate de p-nitrophényle (2 eq). On laisse le mélange réactionnel sous agitation à RT pendant 24 h. On concentre ensuite le milieu réactionnel par distillation de HMPT sous pression réduite. Le résidu d'évaporation est repris plusieurs fois avec une solution aqueuse de AcOH à 30 % p/v. La solution acétique est purifiée par HPLC préparative sur un système "PROTEIN-PAK" ® (colonne échangeuse d'ions "SP 5PW" de dimensions : 15cm x 2,15cm; commercialisée par la société dite MILLIPORE WATERS). On obtient le produit attendu à l'état pur avec un rendement de 45% :
$[\alpha]^{20}_D$ = -10,9° (c = 1% dans MeOH)
Ce produit ainsi obtenu est homogène en CCM :
Rf. = 0,6 avec comme éluant : $CHCl_3$-MeOH-AcOH (5/3/1) v/v; et
Rf. = 0,5 avec comme éluant : $CHCl_3$-MeOH-AcOH-$H_2O$ (60/45/24/6) v/v.
Les fractions obtenues après chromatographie préparative sont contrôlées en HPLC sur une colonne de même nature (de dimensions : 7,5cm x 0,75 cm), la phase mobile utilisée étant un gradient de 2 tampons allant en 25 minutes de 100 % de tampon A
(Tris 20 mM à pH 8,5 et acétonitrile 10 %) à 100 % de tampon B (Tris 20 mM à pH 8,5 + NaCl 1 M et acétonitrile 10 %) à un débit de 1 ml/min, la détection étant réalisée à 254 nm et à 20°C.

b) H-Arg-pNA, 2HBr

A 10 g (23 mmoles) de Z-Arg-pNA, HCl ainsi obtenu on ajoute successivement 20 ml d'anisole. 80 ml de AcOH glacial et 100 ml d'une solution de HBr a 45 % dans AcOH glacial. On laisse le milieu réactionnel à RT pendant une heure et suit l'évolution de la réaction par CCM. Dès qu'elle est achevée, on transvase le milieu réactionnel dans un litre d'éther et agite vigoureusement pendant quelques minutes. Après décantation, le surnageant est aspiré au moyen d'un tube plongeant muni d'un filtre en verre fritté. L'opération de lavage dans l'éther est répétée plusieurs fois (au moins trois fois) et le produit obtenu est séché. On obtient 9,5 g (rendement : 97 %) de H-Arg-pNA, 2HBr sous forme de poudre $[\alpha]^{20°C}_{546\ nm}$ = +55,1° (c = 1% dans MeOH).
Ce produit est homogène en CCM [ éluant : $CHCl_3$/MeOH/AcOH (5/3/1) v/v]; Rf. = 0,4.
L'analyse par HPLC sur "PROTEIN-PAK" "SP 5PW" (dimensions : 7,5 cm x 0,75 cm) avec le gradient sus-décrit de tampons A et B donne un temps de rétention de 34,6 minutes.

c) Boc-L-4Hyp-L-Arg-pNA, HBr

On introduit sous agitation 10 g (1 eq) de H-L-Arg-pNA, 2HBr et 5,0575 g (1 eq) de Boc-L-4Hyp-OH dans un ballon contenant 66 ml de DMF et refroidi au moyen d'un bain de glace. Lorsque la température d'équilibre est atteinte, on ajoute 3,055 ml (1 eq) de $ET_3N$ 6,785 g (1,5 eq) de DCCI et 6,695 g (2eq) de HOBT. On laisse réagir le milieu réactionnel pendant 24 heures. On contrôle l'achèvement de la réaction par CCM puis ajoute la réaction terminée 10 ml de AcOH glacial et agite pendant 0,25 h. Par filtration de la DCHU formée et évaporation du filtrat sous pression réduite, on obtient par précipitation dans l'éther 6,823 g du produit attendu homogène par CCM et HPLC.

**Analyse**

1°) CCM sur couche de silice
- CCM (A) avec éluant : mélange $CHCl_3$/MeOH/AcOH (50/30/10) v/v

Rf = 0,75
- CCM (B) avec éluant : mélange n-butanol/AcOH/eau (3/1/1) v/v
Rf = 0,60
2°) HPLC sur "PROTEIN PAK" (avec gradient de deux tampons comme indiqué ci-dessus : les tampons A et B précités), temps de rétention 16,6 minutes.
3°) Pouvoir rotatoire
$[\alpha]^{20°C}_{546\ nm}$ = -45,50° (c = 1% dans MeOH)

d) H-L-4Hyp-L-Arg-pNA, 2H-TFA

Dans un ballon muni d'une garde de $CaCl_2$, on introduit 6,823 g (1 eq) de Boc-L-4Hyp-L-Arg-pNA, HBr, 32,6 ml (3,7 eq) de H-TFA et 32,6 ml de dichlorométhane. La durée de réaction est d'environ 1 h; 30 min après le début de la réaction, on contrôle son évolution par CCM (A) et CCM (B) comme indiqué ci-dessus au stade c). Lorsque la réaction est achevée, on évapore H-TFA sous pression réduite, puis par précipitation dans l'éther, on obtient 7,382 g du produit attendu.

**Analyse**

1°) CCM sur silice
CCM (A) :     Rf = 0.55
CCM (B) :     Rf = 0,42
2°) HPLC (avec gradient de deux tampons : tampon A comme au stade b) et tampon C (Tris 20mM à pH 8,5 + acétonitrile 10 % + NaCl 1M)), temps de rétention : 18,4 minutes.
3°) $[\alpha]^{20°C}_{546\ nm}$ = -21,4° (c = 1% dans MeOH).

e) MM-L-4Hyp-L-Arg-pNA, H--TFA

On introduit sous agitation 7, 382 g (12 mmoles) de H-L-4Hyp-L-Arg-pNA, 2H-TFA avec 35 ml de DMF dans un ballon de 50 ml refroidi au bain de glace et maintient l'agitation. Lorsque la température d'équilibre est atteinte, on ajoute 4,4 ml (31,4 mmoles; soit 2,7 eq) de $Et_3N$ puis 2,5 ml (24 mmoles) de chlorure de méthyloxymalonyle (MMCl). Après 6 h on contrôle l'achèvement de la réaction par CCM. Si la réaction n'est pas terminée on ajoute 1,5 eq (2,5 ml) de $Et_3N$ puis 5 minutes après 1 eq (1,25 ml) de MMCl. Au bout de 24 h on contrôle l'achèvement de la réaction par CCM. La réaction étant achevée on évapore à sec sous pression réduite et par précipitation dans l'éther on obtient 7,219 g du produit attendu. Ce produit brut est purifié par HPLC préparative et contrôlé par HPLC analytique. Seules sont réunies les fractions homogènes répondant favorablement à une hydrolyse enzymatique et conduisant à une libération de pNa. Les fractions ainsi retenues que l'on obtient sont concentrées sous pression réduite puis lyophilisées.

**Analyse**

1°) CCM sur silice
CCM (A) :     Rf = 0,85
CCM (B) :     Rf = 0,52
2°) HPLC (avec un gradient de deux tampons: tampons A et B du stade b)), temps de rétention : 16 minutes.
3°) $[\alpha]^{20°C}_{546\ nm}$ = -30,4° (c = 1 % dans MeOH)

**PREPARATION II**

En procèdant suivant le procédé de la préparation I, mais en remplaçant l'isocyanate de p-nitrophényle par l'isocyanate de $\sigma$-chloro-p-nitrophényle, on obtient le MM-L-4Hyp-L-Arg-(o-Cl)-pNA, H-TFA.

**PREPARATION III**

Obtention de MM-L-Pro-L-Arg-pNA, H-TFA (Exemple 1; n° de code : SQ 41)
Dans un ballon on dissout successivement 317 mg (2,68 mmoles) d'acide monométhyloxymalonique, 1,66 g (2,68 mmoles) de H-L-Pro-L-Arg-pNA, 2H-TFA, 800 mg (5,36 mmoles) de HOBT et 413$\mu$l (2,95 mmoles) de $Et_3N$ dans 15 ml de DMF. Le mélange est refroidi dans de la glace et mis sous agitation. On ajoute 660 mg (3,2 mmoles) de DCCI et maintient l'agitation pendant une heure à 0°C et 24 heures à RT. On suit l'évolution de la réaction par CCM. Après achèvement de la réaction on filtre pour écarter la DCHU formée et évapore le solvant du filtrat sous pression réduite.Le résidu d'évaporation ainsi obtenu est précipité dans l'éther et purifié par HPLC preparative sur dispositif "PROTEIN PAK" SP 5 PW ( de dimensions 15 cm x 2,15 cm). Les fractions recueillies sont homogènes en CCM et HPLC.

**Analyse**

1°) CCM sur silice
    CCM (A) :     Rf = 0,64
    CCM (B) :     Rf = 0,60
2°) HPLC (gradient de deux tampons : tampons A et B de la préparation Ia)), temps de rétention : 16,4 minutes
3°) $[\alpha]^{20°C}_{546\ nm}$ = -65,9° (c = 1% dans MeOH)
 On a consigné ci-après dans le tableau II les caractéristiques des produits suivant l'invention.

TABLEAU II

| PRODUIT | $[\alpha]^{20°C}_{546\ nm}$ (conc./MeOH) | Rf CCM(A) | CCM(B) | HPLC (a) | (b) |
|---------|------------------------------------------|-----------|--------|----------|-----|
| EX 1 | -65,9° (1%) | 0,64 | 0,60 | 16,4 | A-B |
| EX 2 | -33° (1%) | 0,98 | 0,64 | 14 | A-C |
| EX 3 | -25° (1%) | 0,96 | 0,56 | 13 | A-C |
| EX 4 | -9,6° (1%) | 0,94 | 0,69 | 34,4 | A-B |
| EX 5 | -16,9° (1%) | 0,97 | 0,75 | 37,5 | A-B |
| EX 6 | -18,7° (1%) | 0,79 | 0,58 | 26,2 | A-B |
| EX 7 | -22° (1%) | 1,0 | 0,66 | 22 | A-B |
| EX 8 | -46,7° (1%) | 0,99 | 0,64 | 26,6 | A-B |
| EX 9 | +21,3° (1%) | 0,975 | 0,63 | 19,8 | A-B |
| EX 10 | -30,4° (1%) | 0,85 | 0,52 | 16 | A-B |
| EX 11 | -13,5° (1%) | 0,8 | 0,5 | 19,9 | A-B |
| EX 12 | -40,4° (1%) | 0,92 | 0,53 | 23,7 | A-B |
| EX 13 | +1,08° (1%) | 0,9 | 0,56 | 23,6 | A-B |
| EX 14 | -15,8° (1%) | 0,925 | 0,59 | 17,1 | A-B |
| EX 15 | -13,0° (1%) | 0,90 | 0,77 | 33,8 | A-B |

Notes:

(a) temps de rétention en minutes

(b) gradient de deux tampons allant en 25 minutes de 100% de A à 100 % de B ou C, puis restant pendant 20 minutes à 100% de B ou C; lecture à 254 nm, à 20° C (pour tampons A et B, voir préparation Ia et pour tampon C voir préparation Id)

## ACTIVITE DES SUBSTRATS

L'activité des dipeptides suivant l'invention et des tripeptides consignés dans le tableau I a été étudiée sur la thrombine humaine, la plasmine humaine, le Facteur Xa humaine et la Protéine C bovine, les tripeptides A1-A4 étant les substrats de référence du commerce. La libération du marquer $HR_1$ (i.e. amine $H_2NR'_1$), qui est objectivée par la variation de OD pendant l'hydrolyse enzymatique des substrats, sert

14

d'outil de comparaison.

La lecture de OD est en général effectuée à 300-470 nm, et plus précisément à 405 nm quand $R_1$ est pNA, pendant environ 5 minutes.

Les essais ont été réalisés sur appareil GILFORD 203 S pour automatiser la lecture des échantillons et standardiser leur traitement.

### Enzymes

On prépare des solutions dans du sérum physiologique renfermant chacune un enzyme :

| | |
|---|---|
| thrombine humaine | 3 UNIH/ml |
| Facteur Xa humain | 3,7 nkat/ml |
| plasmine humaine | 2,3 nkat/ml |
| protéine C bovine activée | 10μg/ml |

### Substrats

On dissout chaque peptide à tester dans le l'eau à une concentration de 10 mg/ml.

### Mesures

400 μl d'enzyme dilué au 1/2 dans du tampon TEA (pH 8,4) sont ajoutés à 200 μl de chacun des différents peptides à tester. On détermine les vitesses d'hydrolyse desdits substrats en suivant l'évolution de la libération de pNA. Cette évolution peut être suivie par la lecture de la varition de OD par unité de temps (ΔOD/minute) à 405 nm.

### Résultats

Les résultats obtenus (exprimés en ΔOD/minute) sont consignés dans le tableau III ci-après. Ils mettent en évidence que :

(i) les produits EX 1 et EX 10 sont particulièrement intéressants en tant que substrats, EX 1 et EX 10 étant plus sensibles à la protéine C que le produit de référence A4, le produit EX 10 étant en outre plus sensible à la plasmine que le produit de référence A3;

(ii) le produit EX 12 est particulièrement spécifique vis-à-vis de la protéine C;

(iii) le produit EX 4 est particulièrement spécifique vis-à-vis de la plasmine;

(iv) le produit EX 11 est plus sensible vis-à-vis du facteur Xa que vis-à-vis de la thrombine, de la plasmine et de la protéine C; et

(v) dans la série des tripeptides, par comparaison de B1 avec C1, de B2 avec C2, de B3 avec C3, de B4 avec C4, on constate que le remlacement du groupe H de l'atome d'azote terminal du peptide par le groupe MM selon l'invention, diminue systématiquement l'activité.

TABLEAU III

| PRODUIT | N°code | Vitesse d'hydrolyse en ΔOD/minute | | | |
|---------|--------|-----------|------------|----------|-------------|
| | | Thrombine | Facteur Xa | Plasmine | Protéine C |
| EX 1 | SQ 41 | 0,68 | 0,14 | 0,230 | 0,83 |
| EX 2 | SQ 57 | 0,21 | 0,03 | 0,08 | 0,12 |
| EX 3 | SQ 58 | 0,20 | 0,05 | 0,03 | 0,04 |
| EX 4 | SQ 59 | 0,05 | 0,13 | 0,64 | 0,16 |
| EX 5 | SQ 60 | 0,07 | 0,09 | 0,20 | 0,08 |
| EX 6 | SQ 61 | 0,02 | 0,03 | 0,05 | 0,02 |
| EX 7 | SQ 62 | 0,04 | 0,16 | 0,17 | 0,10 |
| EX 8 | SQ 63 | 0,04 | 0,16 | 0,21 | 0,11 |
| EX 9 | SQ 64 | 0,14 | 0,07 | 0,17 | 0,12 |
| EX 10 | SQ 65 | 0,61 | 0,34 | 1,12 | 0,88 |
| EX 11 | SQ 66 | 0,03 | 0,15 | 0,04 | 0,04 |
| EX 12 | SQ 67 | 0,18 | 0,07 | 0,14 | 0,41 |
| EX 13 | SQ 68 | 0,04 | 0,01 | 0,02 | 0,03 |
| EX 14 | SQ 69 | 0,19 | 0,06 | 0,10 | 0,25 |
| EX 15 | SQ 70 | 0,05 | 0,01 | 0,04 | 0,08 |
| EX 16 | SQ 73 | 0,24 | 0,03 | 0,07 | 0,08 |
| A1 | 34.47 | 0,782 | - | - | - |
| A2 | 31.39 | - | 1,312 | - | - |
| A3 | 30.41 | - | - | 0,235 | - |
| A4 | 65,25 | - | - | - | 0,520 |

## TABLEAU III (fin)

| | | | | | |
|---|---|---|---|---|---|
| B1 | SQ 72 | 0,13 | 0,02 | 0,03 | 0,18 |
| C1 | – | 0,516 | 0,01 | 0,04 | 0,24 |
| B2 | SQ 71 | 0,14 | 0,01 | 0,04 | 0,09 |
| C2 | – | 0,35 | 0,04 | 0,02 | 0,17 |
| B3 | SQ 56 | 0,05 | 0,01 | 0,01 | 0,03 |
| C3 | 55.10 | 0,32 | 0,59 | 0,02 | 1,08 |
| B4 | SQ 52 | 0,01 | 0,01 | 0,04 | 0,02 |
| C4 | 33.08 | 0,61 | 0,02 | 0,65 | 0,11 |

Les dérivés dipeptides de formule I selon l'invention sont particulièrement intéressants en tant que substrats enzymatiques dans le domaine des dosages. Ils peuvent être conditionnés sous forme de kits ou trousses de dosage avec les autres réactifs requis pour lesdits dosages.

**Revendications**

1. Composé dipeptidique caractérisé en ce qu'il est choisi parmi l'ensemble comprenant :
    (i) les composés de formule :

    $Q-A_1-A_2-R_1$     (I)

    dans laquelle :
    Q est un reste $RO-CO-CH_2-CO$ où
    R représente l'atome d'hydrogène, un groupe alkyle en $C_1-C_4$, un groupe phényle éventuellement substitué par un ou plusieurs groupes $CH_3$ et $OCH_3$, un groupe 3,4-méthylènedioxyphényle, un groupe benzyle éventuellement substitué par un ou plusieurs groupes $CH_3$ et $OCH_3$, un groupe 3,4-méthylènedioxybenzyle, ou, un groupe tosylméthyle;
    $R_1$ qui est un reste amino de formule $NH-R'_1$ constitue un marqueur clivable du groupe $A_2$ par hydrolyse enzymatique, le groupe $R'_1$ intervenant comme support du moyen de marquage;
    $A_1$ est un reste de monoaminoacide choisi parmi l'ensemble comprenant les restes d'aminoacides non-basiques;
    $A_2$ est un reste de monoaminoacide choisi parmi l'ensemble comprenant les restes d'α-aminoacides basiques; et
    (ii) leurs sels d'addition.

2. Composé dipeptidique suivant la revendication 1, caractérisé en ce que $A_1$ est un reste choisi parmi l'ensemble comprenant
    1°) les restes d'aminoacides naturels ou synthétiques qui comprennent une seule fonction acide et une seule fonction basique,
    2°) les restes d'aminoacides naturels ou synthétiques qui comprennent une seule fonction basique et plus d'une fonction acide et dans lesquels chaque fonction acide latérale est susceptible d'être bloquée notamment sous forme amide ou ester, et
    3°) les restes d'aminoacides naturels ou synthétiques qui comprennent une seule fonction acide et plus d'une fonction basique et dans lesquels chaque fonction basique latérale est convenablement substituée pour supprimer essentiellement le caractère basique,

17

lesdits restes sus-visés pouvant comporter des groupes hydroxyle (OH) ou thiol (SH) latéraux qui sont susceptibles d'être bloqués, le cas échéant, par un groupe protecteur éther ou ester.

**3.** Composé dipeptidique suivant la revendication 1, caractérisé en ce que $A_1$ est choisi parmi l'ensemble constitué par

a) Ala, bAla, Cys, Gln, Gly, 4Hyp, 3Hyp, Leu, Met, Nle, Nva, Phe, Pro, MeGly, Ser, Thr, Tyr et Val, où le groupe OH latéral des restes 4Hyp, 3Hyp, Ser, Thr et Tyr est protégé ou non par un groupe protecteur éther ou ester, et où le groupe SH latéral du reste Cys est protégé ou non par un groupe protecteur thioéther ou thioester;

b) Asp, bAsp, Glu et $\gamma$-glutaminyle, dans lesquels la fonction acide latérale est libre ou bloquée sous forme ester, notamment sous forme d'ester benzylique ou t-butylique;

c) Arg, Dbu, Lys, His et Orn convenablement bloqué pour éliminer substantiellement le caractère basique;

d) 1-amino-cyclohexane-1-carbonyle (ACC), Acp, Aib, thiazolidine-4-carbonyle (ATC), azétidine-2-carbonyle (Aze), Abu, 4Abu, 3-cyclohexylalanyle (CHA), $\alpha$-cyclohexylglycyle (CHG), 3-(4-hydroxycyclohexyl)alanyle (CHT) où le groupe OH latéral est protégé le cas échéant sous forme éther ou ester, cycloleucyle (Cle), pyrodiaminobutyryle, $\alpha$-phénylglycyle, Pip et pyroglutaminyle (Pyr).

**4.** Composé dipeptidique suivant la revendication 1, caractérisé en ce que le groupe $A_1$ est choisi parmi l'ensemble comprenant les restes Aib, L-Ala, bAla, L-thiazolidine-4-carbonyle (L-ATC), L-azétidine-2-carbonyle (L-Aze), L-Abu, 4Abu, L-3-cyclohexylalanyle (L-CHA), L-$\alpha$-cyclohexylglycyle (L-CHG), L-3-(4-hydroxycyclohexyl)-alanyle (L-CHT), Cle, Gly, L-4Hyp, L-3Hyp, L-Ile, L-Leu, L-Nle, L-Nva, L-Phe, L-Pip, L-Pro, MeGly, L-Ser, L-Thr, L-Tyr et L-Val, les restes L-3-(4-hydroxycyclohexyl)alanyle (L-CHT), L-4Hyp, L-3Hyp, L-Ser, L-Thr et L-Tyr dans lesquels le groupe OH latéral est protégé par un groupe éther ou ester, et, les restes L-Arg, L-Lys, L-His et L-Orn dans lesquels la fonction basique latérale est convenablement bloquée pour éliminer substantiellement le caractère basique.

**5.** Composé dipeptidique suivant la revendication 1, caractérisé en ce que $A_2$ est choisi parmi l'ensemble comprenant les restes L-Arg, L-Lys, L-His et L-Orn.

**6.** Composé dipeptidique suivant la revendication 1, caractérisé en ce que R est $CH_3$.

**7.** Composé dipeptidique suivant la revendication 1, caractérisé en ce que le groupe $R_1$ est choisi parmi les groupes aminés NH-R', qui (i) induisent une modification de coloration et (ii) induisent une modification de fluorescence ou (iii) comportent au moins un élément radioactif.

**8.** Composé dipeptidique suivant la revendication 7, caractérisé en ce que $R_1$ est pNA.

**9.** Composé dipeptidique suivant la revendication 1, caractérisé en ce qu'il est choisi parmi l'ensemble comprenant les composés :

$CH_3O$-CO-$CH_2$-CO-L-Pro-L-Arg-pNA,
$CH_3O$-CO-$CH_2$-CO-L-4Hyp-L-Arg-pNA,

et leurs sels d'addition.

**10.** Procédé de préparation d'un composé dipeptidique de formule I suivant la revendication 1, caractérisé en ce qu'il comprend :

1°) la réaction d'un $\alpha$-aminoacide N-protégé par un groupe protecteur temporaire approprié $Z_1$ de formule :

$Z_1$-$A_2$-OH      (III)

où $A_2$ est défini comme ci-dessus, avec un isocyanate, correspondant au groupe marqueur $R_1$ de formule :

O = C = N-R'$_1$      (IV)

pour obtenir un composé de formule :

$Z_1$-$A_2$-$R_1$    (V)

2°) la soumission du composé V ainsi obtenu à une réaction d'acidolyse au moyen d'un mélange HBr/AcOH pour éliminer le groupe protecteur $Z_1$ et obtenir le dérivé de formule :

H-$A_2$-$R_1$    (VI)

3°) la réaction du dérivé VI ainsi obtenu avec un aminoacide N-protégé de formule :

$Z_2$-$A_1$-OH    (VII)

où $A_1$ est défini comme indiqué ci-dessus et $Z_2$ est un groupe protecteur temporaire analogue à $Z_1$, pour obtenir par condensation un dipeptide protégé de formule :

$Z_2$-$A_1$-$A_2$-$R_1$    (VIII)

4°) la soumission dudit dipeptide N-protégé de formule VIII ainsi obtenu à une réaction d'acidolyse au moyen de l'acide trifluoroacétique pour éliminer le groupe protecteur $Z_2$ et donner un dipeptide de formule :

H-$A_1$-$A_2$-$R_1$    (IX)

et,

5°) la réaction du dipeptide de formule IX ainsi obtenu avec un composé de formule :

R-O-CO-$CH_2$-CO-T    (X)

où R est défini comme indiqué ci-dessus et T représente OH, F, Cl ou Br, pour obtenir le composé de formule I attendu.

**Claims**

1. A dipeptide compound characterized in that it is selected from the group consisting of
    (i) compounds of the formula

    Q-$A_1$-$A_2$-$R_1$    (I)

    wherein
    Q is an oxymalonyl group R-O-CO-$CH_2$-CO in which
    R is a hydrogen atom, a $C_1$-$C_4$ alkyl group, a phenyl group optionally substituted by one or several groups $CH_3$ and $OCH_3$, a 3,4-methylenedioxyphenyl group, a benzyl group optionally substituted by one or several groups $CH_3$ and $OCH_3$, a 3,4-methylenedioxybenzyl group, or a tosylmethyl group ;
    $R_1$, which is an amino radical NR-$R'_1$, constitutes a label cleavable from $A_2$ by enzymatic hydrolysis, the group $R'_1$ serving as a carrier for the labeling means ;
    $A_1$ is a monoamino acid residue selected from the group comprising non-basic amino acid residues ;
    $A_2$ is a monoamino acid residue selected from the group comprising basic $\alpha$-amino acid residues ;
    and
    (ii) addition salts thereof.

2. A dipeptide compound according to claim 1, wherein $A_1$ is selected from the group consisting of
    1°) residues of natural and synthetic monoamino acids containing only one acid group and only one basic group,
    2°) residues of natural and synthetic monamino acids containing only one basic group and more than one acid group in which each acid side-group can be blocked, especially in the form of an ester or amid, and
    3°) residues of natural and synthetic monoamino acids containing only one acid group and more

19

than one basic group in which each basic side-group is appropriately substituted so as substantially to eliminate the basic character,

it being possible for the said residues mentioned above to contain hydroxyl (OH) or thiol (SH) side-groups which are capable of being blocked, if necessary, by an ether or ester protecting group.

3. A dipeptide compound according to claim 1, wherein $A_1$ is selected from the group consisting of :

a) Ala, bAla, Cys, Gln, Gly, 4Hyp, 3Hyp, Leu, MeGly, Met, Nle, Nva, Phe, Pro, Ser, Thr, Tyr and Val in which the OH side-group of 4Hyp, 3Hyp, Ser, Thr and Tyr is or is not protected by an ether or ester protecting group, and in which the SH side-group of Cys is or is not protected by a thioether or thioester protecting group ;

b) Asp, bAsp, Asp, Glu and $\gamma$-glutaminyl in which the acid side-group is free or blocked in the form of an ester, especially in the form of a benzyl or t-butyl ester ;

c) Arg, Dbu, Lys, His and Orn appropriately blocked so as substantially to eliminate the basic character ; and

d) Abu, 4Abu, 1-aminocyclohexane-1-carbonyl (ACC), Acp, Aib, thiazolidine-4-carbonyl (ATC), azetidine-2-carbonyl (Aze), 3-cyclohexylalanyl (CHA), $\alpha$-cyclohexylglycyl (CHG), 3-(4-hydroxycyclohexyl)alanyl (CHT), pyrodiaminobutyryl, cycloleucyl (Cle), $\alpha$-phenylglycyl (Phg), Pip and pyroglutaminyl (Pyr), in which the OH side-group of CHT can be protected by an ether or ester protecting group.

4. A dipeptide compound according to claim 1, wherein $A_1$ is selected from the group consisting of the residues Aib, L-Ala, bAla, L-1-aminocyclohexane-1-carbonyl (L-Aze), L-thiazolidine-4-carbonyl (L-ATC), L-Abu, 4Abu, L-3-cyclohexylalanyl (L-CHA), L-$\alpha$-cyclohexylglycyl (L-CHG), 3-(4-hydroxycyclohexyl)-alanyl (L-CHT), Cle, Gly, L-4Hyp, L-3Hyp, L-Ile, L-Leu, L-Nle, L-Nva, L-Phe, L-Pip, L-Pro, MeGly, L-Ser, L-Thr, L-Tyr and L-Val, the residues L-3-(4-hydroxycyclohexyl)alanyl (L-CHT), L-4Hyp, L-3Hyp, L-Ser, L-Thr and L-Tyr in which the OH side-group is protected by an ether or ester protecting group, and, the residues L-Arg, L-Lys, L-His and L-Orn in which the basic side-group is appropriately blocked so as substantially to eliminate the basic character.

5. A dipeptide compound according to claim 1, wherein $A_2$ is selected from the group consisting of L-Arg, L-Lys, L-His and L-Orn.

6. A dipeptide compound according to claim 1, wherein R is Me.

7. A dipeptide compound as claimed in claim 1, wherein the group $R_1$ is selected from the amine groups NH-$R'_1$ which (i) induce a color modification, (ii) induce a fluorescence modification or (iii) contain at least one radioactive element.

8. A dipeptide compound according to claim 7, wherein $R_1$ is pNA.

9. A dipeptide compound according to claim 1, which is selected from the group consisting the compounds

MeO-CO-CH$_2$-CO-L-4Hyp-L-Arg-pNA,
MeO-CO-CH$_2$-CO-L-Pro-L-Arg-pNA, and

addition salts thereof.

10. A method for the preparation of a dipeptide compound of the formula I as claimed in claim 1, which comprises :

1) reacting an $\alpha$-amino acid N-protected by an appropriate temporary protecting group $Z_1$, of the formula :

Z$_1$A$_2$-OH    (III)

in which $A_2$ is defined as above, with an isocyanate corresponding to the labelling group $R_1$, of the formula :

$$O = C = N\text{-}R'_1 \qquad (IV)$$

to give a compound of the formula :

$$Z_1\text{-}A_2\text{-}R_1 \qquad (V)$$

2) subjecting the resulting compound V to an acidolysis reaction by means of an HBr/AcOH mixture to remove the protecting group $Z_1$ and give the derivative of the formula:

$$H\text{-}A_2\text{-}R_1 \qquad (VI)$$

3) reacting the resulting derivative VI with an N-protected amino acid of the formula :

$$Z_2\text{-}A_1\text{-}OH \qquad (VII)$$

in which $A_1$ is defined as indicated above and $Z_2$ is a temporary protecting group analogous to $Z_1$, to give a protected dipeptide of the formula :

$$Z_2\text{-}A_1\text{-}A_2\text{-}R_1 \qquad (VIII)$$

by condensation,
4) subjecting the resulting N-protected dipeptide of the formula VIII to an acidolysis reaction by means of trifluoroacetic acid to remove the protecting group $Z_2$ and give a dipeptide of the formula :

$$H\text{-}A_1\text{-}A_2\text{-}R_1 \qquad (IX)$$

and
5) reacting the resulting dipeptide of the formula IX with a compound of the formula :

$$R\text{-}O\text{-}CO\text{-}CH_2\text{-}CO\text{-}T \qquad (X)$$

in which R is defined as indicated above and T represents OH, F, Cl or Br, for obtaining the expected compound of the formula I.

**Patentansprüche**

1. Dipeptidverbindung, dadurch gekennzeichnet, daß sie aus der aus
   (i) Verbindungen der Formel

   $$Q\text{-}A_1\text{-}A_2\text{-}R_1 \qquad (I)$$

   worin Q ein Rest $RO\text{-}CO\text{-}CH_2\text{-}CO$ ist, worin
   
   R ein Wasserstoffatom, eine $C_1\text{-}C_4$-Alkylgruppe, eine gegebenenfalls durch eine oder mehrere $CH_3$- und $OCH_3$-Gruppen substituierte Phenylgruppe, eine 3,4-Methylendioxyphenylgruppe, eine gegebenenfalls durch ein oder mehrere $CH_3$- und $OCH_3$-Gruppen substituierte Benzylgruppe, eine 3,4-Methylendioxybenzylgruppe oder eine Tosylmethylgruppe darstellt;
   
   $R_1$, das ein Aminorest der Formel $NH\text{-}R'_1$ ist, eine von der Gruppe $A_2$ durch enzymatische Hydrolyse abspaltbare Markierung bildet, wobei die Gruppe $R'_1$ zur Unterstützung des Markierungsmittels dient;
   
   $A_1$ ein Monoaminosäurerest ist, ausgewählt aus der aus nicht basischen Aminosäureresten bestehenden Gruppe;
   
   $A_2$ ein Monoaminosäurerest ist, ausgewählt aus der aus basischen $\alpha$-Aminosäureresten bestehenden Gruppe und
   
   (ii) deren Additionssalzen bestehenden Gruppe ausgewählt sind.

2. Dipeptidverbindung nach Anspruch 1, dadurch gekennzeichnet, daß $A_1$ ein aus der aus
   1) natürlichen oder synthetischen Aminosäureresten, die eine einzige Säurefunktion und eine einzige

EP 0 280 610 B1

Basenfunktion enthalten,

2) natürlichen oder synthetischen Aminosäureresten, die eine einzige Basenfunktion und mehr als eine Säurefunktion enthalten und worin jede laterale Säurefunktion insbesondere in Form eines Amids oder Esters blockiert werden kann, und

3) natürlichen oder synthetischen Aminosäureresten, die eine einzige Säurefunktion und mehr als eine Basenfunktion enthalten und worin jede laterale Basenfunktion geeignet substituiert ist, um den basischen Charakter im wesentlichen zu unterdrücken,

bestehenden Gruppe ausgewählter Rest ist, wobei die vorstehend genannten Reste laterale Hydroxylgruppen (OH) oder Thiolgruppen (SH) enthalten können, die gegebenenfalls mit einer Ether- oder Esterschutzgruppe blockiert werden können.

3. Dipeptidzusammensetzung nach Anspruch 1, dadurch gekennzeichnet, daß $A_1$ aus der aus

a) Ala, bAla, Cys, Gln, Gly, 4Hyp, 3Hyp, Leu, Met, Nle, Nva, Phe, Pro, MeGly, Ser, Thr, Tyr und Val, worin die laterale OH-Gruppe der Reste 4Hyp, 3Hyp, Ser, Thr und Tyr durch eine Ether- oder Esterschutzgruppe geschützt ist oder auch nicht und worin die laterale SH-Gruppe des Rests Cys durch eine Thioether- oder Thioester-Schutzgruppe geschützt ist oder auch nicht;

b) Asp, bAsp, Glu und γ-Glutaminyl, worin die laterale Säurefunktion frei oder in Form eines Esters, insbesondere in Form eines Benzyl- oder t-Butylesters, blockiert ist;

c) Arg, Dbu, Lys, His und Orn, die geeignet blockiert sind, um den basischen Charakter im wesentlichen zu eliminieren;

d) 1-Aminocyclohexan-1-carbonyl (ACC), Acp, Aib, Thiazolidin-4-carbonyl (ATC), Azetidin-2-carbonyl (Aze), Abu, 4Abu, 3-Cyclohexylalanyl (CHA), α-Cyclohexylglycyl (CHG), 3-(4-Hydroxycyclohexyl)-alanyl (CHT), worin die laterale OH-Gruppe gegebenenfalls in Form eines Ethers oder Esters geschützt sein kann, Cycloleucyl (Cle), Pyrodiaminobutyryl, α-Phenylglycyl, Pip und Pyroglutaminyl (Pyr) gebildeten Gruppe ausgewählt ist.

4. Dipeptidverbindung nach Anspruch 1, dadurch gekennzeichnet, daß die Gruppe $A_1$ aus der die Reste Aib, L-Ala, bAla, L-Thiazolidin-4-carbonyl (L-ATC), L-Azetidin-2-carbonyl (L-Aze), L-Abu, 4Abu, L-3-Cyclohexylalanyl (L-CHA), L-α-Cyclohexylglycyl (L-CHG), L-3-(4-Hydroxycyclohexyl)alanyl (L-CHT), Cle, Gly, L-4Hyp, L-3Hyp, L-Ile, L-Leu, L-Nle, L-Nva, L-Phe, L-Pip, L-Pro, MeGly, L-Ser, L-Thr, L-Tyr und L-Val umfassenden Gruppe ausgewählt ist, wobei in den Resten L-3-(4-Hydroxylcyclohexyl)alanyl (L-CHT), L-4Hyp, L-3Hyp, L-Ser, L-Thr und L-Tyr die laterale OH-Gruppe durch eine Ether- oder Estergruppe geschützt ist und in den Resten L-Arg, L-Lys, L-His und L-Orn die laterale Basenfunktion geeignet blockiert ist, um den basischen Charakter im wesentlichen zu eliminieren.

5. Dipeptidverbindung nach Anspruch 1, dadurch gekennzeichnet, daß $A_2$ aus der die Reste L-Arg, L-Lys, L-His und L-Orn umfassenden Gruppe ausgewählt ist.

6. Dipeptidverbindung nach Anspruch 1, dadurch gekennzeichnet, daß R $CH_3$ ist.

7. Dipeptidverbindung nach Anspruch 1, dadurch gekennzeichnet, daß die Gruppe $R_1$ aus den aminierten Gruppen NH-R' ausgewählt ist, die (i) eine Farbänderung induzieren und (ii) eine Fluoreszenzänderung induzieren oder (iii) wenigstens ein radioaktives Element tragen.

8. Dipeptidverbindung nach Anspruch 7, dadurch gekennzeichnet, daß $R_1$ pNA ist.

9. Dipeptidverbindung nach Anspruch 1, dadurch gekennzeichnet, daß sie aus der die Verbindungen

$CH_3O-CO-CH_2-CO-L-Pro-L-Arg-pNA$,

$CH_3O-CO-CH_2-CO-L-4Hyp-L-Arg-pNA$

und deren Additionssalze umfassenden Gruppe ausgewählt ist.

10. Verfahren zur Herstellung einer Dipeptidverbindung der Formel I nach Anspruch 1, dadurch gekennzeichnet, daß es

1) die Reaktion einer α-Aminosäure, die durch eine geeignete zeitweilige Schutzgruppe $Z_1$ der Formel

22

$Z_1$-$A^2$-OH      (III)

N-geschützt ist, worin $A_2$ wie vorstehend definiert ist, mit einem Isocyanat entsprechend der Markierungsgruppe $R_1$ der Formel

$O = C = N$-$R'_1$      (IV)

zwecks Erhalt einer Verbindung der Formel

$Z_1$-$A_2$-$R_1$      (V)

2) die Unterwerfung der so erhaltenen Verbindung V einer azidolytischen Reaktion mit Hilfe einer Mischung aus HBr/AcOH zur Eliminierung der Schutzgruppe $Z_1$ zwecks Erhalt des Derivats der Formel

H-$A_2$-$R_1$      (VI)

3) die Reaktion des so erhaltenen Derivats VI mit einer N-geschützten Aminosäure der Formel

$Z_2$-$A_1$-OH      (VII)

worin $A_1$ definiert ist, wie vorstehend angegeben, und $Z_2$ eine zu $Z_1$ analoge zeitweilige Schutzgruppe ist, zwecks Erhalt eines geschützten Dipeptids der Formel

$Z_2$-$A_1$-$A_2$-$R_1$      (VIII)

durch Kondensation;
4) die Unterwerfung des so erhaltenen N-geschützten Peptids der Formel VIII einer azidolytischen Reaktion mit Hilfe von Trifluoressigsäure zur Eliminierung der Schutzgruppe $Z_2$ unter Erhalt eines Dipeptids der Formel

H-$A_1$-$A_2$-$R_1$      (IX)

und
5) die Reaktion des so erhaltenen Dipeptids der Formel IX mit einer Verbindung der Formel

R-O-CO-$CH_2$-CO-T      (X)

worin R definiert ist, wie vorstehend angegeben, und T OH, F, Cl oder Br darstellt, zwecks Erhalt der erwarteten Verbindung der Formel I umfaßt.